# EUROPEAN PATENT APPLICATION

(11) **EP 2 204 216 A1**
(43) Date of publication of application: **07.07.2010**
(21) Application number: 09181045.7
(22) Date of filing: 31.12.2009
(51) Int. Cl.: A61M 39/28

(54) **Catheter clamping assemblies**

(30) Priority: 31.12.2008 US 141731 P
(71) Applicant: Tyco Healthcare Group LP, Mansfield, MA 02048 (US)
(72) Inventor: BRAGA, Richard, Massachusetts 02356 (US); SANSOUCY, Michael, Massachusetts 02093 (US)
(74) Representative: Gray, James

(57) **Abstract**

A clamping assembly (100) includes a housing (102) and at least one clamp member (104) pivotally coupled to the housing. The housing defines a throughbore (108) which is dimensioned to receive a tubular member (110). The at least one clamp member is configured to move between a first position in which the clamp member is positioned externally of the throughbore to allow fluid to flow through a tubular member positioned within the throughbore and a second position in which the clamp member extends into the throughbore and into contact with a tubular member to obstruct fluid flow through the tubular member positioned within the throughbore.

## Description

### CROSS-REFERENCE TO RELATED APPLICATIONS

The present application claims the benefit of and priority to U.S. Provisional Patent Application Serial No. 61/141,731, filed on December 31, 2008, entitled "Catheter Clamping Assemblies," the entire contents of which are hereby incorporated herein by reference.

### TECHNICAL FIELD

The present disclosure relates generally to clamping assemblies for clamping flexible fluid conduits. More particularly, the present disclosure relates to clamping assemblies for obstructing fluid flow through extension tubes of a catheter assembly.

### BACKGROUND

Catheters are flexible medical devices which facilitate the introduction and withdrawal of fluids to and from body cavities, ducts, and vessels. Catheters may have particular application in a hemodialysis procedure where blood is withdrawn from a blood vessel for treatment, and subsequently returned to the blood vessel for circulation. Known hemodialysis catheters include multiple lumens, e.g., dual lumen catheters which permit bi-directional fluid flow within the catheter whereby one lumen is dedicated for delivery of blood from a body vessel to a blood treatment device, e.g., a dialyzer, and the other lumen is dedicated for returning the treated blood to the vessel. During an exemplary hemodialysis procedure, a multiple lumen catheter is inserted into a body and blood is withdrawn through an arterial lumen of the catheter. The removed blood is directed to a hemodialysis unit which dialyzes, or purifies, the blood to remove waste and toxins from the blood. The dialyzed blood is returned to the patient through a venous lumen of the catheter.

Various techniques and apparatus are employed for the insertion of hemodialysis catheters including, e.g., guidewires, introduction stylets or the like. Some of these known techniques include subcutaneous tunneling methodologies where a subcutaneous tunnel is formed between two spaced openings in the skin with the use of a trocar or the like. One catheter end is introduced through an entry site or venotomy site for routing into, e.g., the jugular vein and routed to the heart. The trailing or proximal end is advanced through the subcutaneous tissue to exit a second exit opening adjacent the sternum of the patient beneath the venotomy site. Once the proximal end of the catheter is exposed, a catheter hub with extension tubes is fluidly connected to the catheter. One subcutaneous technique is disclosed in U.S. Patent No. 5,509,897 to Twardowski et al., the contents of which are incorporated herein by reference in its entirety.

The use of an extension tube assembly including one or more extension tubes and a clamp for obstructing fluid flow through each extension tube is well known in the art. Typically, in a dual lumen catheter, the extension tube assembly includes a first extension tube which fluidly connects the venous lumen of the catheter to the hemodialysis unit and a second extension tube which fluidly connects the arterial lumen of the catheter to the hemodialysis unit. A clamp is positioned about each extension tube to facilitate control of fluid through each extension tube.

Chronic hemodialysis catheters are positioned within a patient for long term use and, thus, may cause discomfort and/or irritation to the patient over extended periods of time. Accordingly, a continuing need exists in the art for an extension tube assembly which minimizes discomfort and/or irritation to a patient and is easy to operate.

### SUMMARY

The present disclosure relates to various embodiments of clamping assemblies for releasably obstructing fluid flow through a tubular member. In one embodiment, the clamping assemblies include a housing and at least one clamp member pivotally coupled to the housing. The housing includes a proximal end portion and a distal end portion, and defines a throughbore which is dimensioned to receive a tubular member. The at least one clamp member is configured to move between a first position in which the clamp member is positioned externally of the throughbore to allow fluid to flow through a tubular member positioned within the throughbore and a second position in which the clamp member extends into the throughbore and into contact with a tubular member to obstruct fluid flow through the tubular member positioned within the throughbore.

In embodiments, the clamp member is pivotally connected to the distal end portion of the housing. In other embodiments, the clamp member is pivotally connected to the proximal end portion of the housing. In yet other embodiments, the clamp member is pivotally connected between the proximal and distal end portions of the housing.

The throughbore may include a central segment extending along a portion of the housing which divides into two branch segments. This configuration accommodates one or more tubular members, such as a y-shaped tubular member. In such embodiments, two clamp members may be pivotally connected to the housing such that each of the branch segments includes one of the two clamp members for independent movement of the clamp members between the first and second positions to allow or obstruct fluid flow through a portion of the tubular member positioned within the respective branch segment of the throughbore.

The clamp assembly may include a clamp actuator for moving the clamp member between the first and second positions. In embodiments, the clamp actuator is monolithically formed with the clamp member. The clamp actuator may include a contoured outer surface to facilitate gripping of the clamp actuator. In embodiments, the clamp actuator is disposed within a slot formed in a sidewall of the housing.

In another embodiment, clamping assemblies include a first clamping section defining a first bore that is dimensioned to receive a tubular member and a second clamping section defining a second bore dimensioned to receive the tubular member. The second clamping section includes at least one engagement member and is configured to be releasably coupled to the first clamping section. The first clamping section is configured to urge the at least one engagement member of the second clamping section to a position at least partially obstructing the second bore when the first clamping section is releasably coupled to the second clamping section.

In yet another embodiment, clamping assemblies include a first housing having a first longitudinal axis and defining a first passageway dimensioned to receive a tubular member and a second housing having a second longitudinal axis and defining a second passageway dimensioned to receive the tubular member. The first and second housings are adapted to pivot between a first position wherein the first and second longitudinal axes are substantially aligned and a second position wherein the first and second longitudinal axes define an angle relative to each other. An engagement member may extend from the second housing along an axis that is substantially parallel to the second longitudinal axis such that the engagement member is positioned to extend at least partially within the first housing to engage and compress the tubular member when the first and second housings are in the second position. A latch mechanism including a first coupling segment and a second coupling segment may be connected to the first housing and the second housing, respectively. At least one of the first and second coupling segments may include at least two contact points for variable positioning of the latch mechanism. The latch mechanism is configured to secure the first and second housings in the first and second positions.

In another embodiment, clamping assemblies include a hub and a locking mechanism. The hub includes a body portion defining at least one throughbore which is dimensioned to receive a tubular member and a base position defining at least one opening dimensioned to releasably receive the tubular member extending through the throughbore in a bent configuration. A locking mechanism includes first and second sleeves which are disposed around the tubular member and configured to secure the tubular member to the base portion while the tubular member is positioned in the bent configuration.

In yet another embodiment, clamping assemblies include a monolithic body including a first clamping member and a second clamping member in juxtaposed relation to each other. The first clamping member defines a first channel that is dimensioned to receive a tubular member. The first clamping member also includes a first movable section, a first fixed section, and a first locking mechanism disposed in mechanical cooperation with the first movable section and the first fixed section. The first movable section is configured to move between open and approximated positions relative to the first fixed section such that the first locking mechanism is configured to releasably retain the first movable section in the approximated position. The second clamping member defines a second channel dimensioned to receive a tubular member. The second clamping member including a second movable section, a second fixed section, and a second locking mechanism disposed in mechanical cooperation with the second movable section and the second fixed section. The second movable section is configured to move between open and approximated positions relative to the second fixed section such that the second locking mechanism is configured to releasably retain the second movable section in the approximated position.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is a side perspective view of an embodiment of the presently disclosed clamping assembly positioned about a tubular member in an open or non-clamped configuration;

FIG. 2 is a side perspective view of the clamping assembly shown in FIG. 1 positioned about a tubular member in a closed or clamped configuration;

FIG. 3 is a side perspective view, with parts shown in phantom, of another embodiment of the presently disclosed clamping assembly positioned about a y-shaped tubular member;

FIG. 4 is a side perspective view, with parts shown in phantom, of yet another embodiment of the present disclosed clamping assembly positioned about a y-shaped tubular member and including a clamp actuator;

FIG. 5 is a side perspective view of another embodiment of the presently disclosed clamping assembly including a clamp actuator;

FIG. 6 is an end perspective view of the clamping assembly shown in FIG. 5 in an open or non-clamped configuration; and

FIG. 7 is an end perspective view of the clamping assembly of FIG. 6 positioned in a closed or clamped configuration.

FIG. 8 is a side perspective view of one embodiment of the presently disclosed clamping assembly with parts separated;

FIG. 9 is a side cross-sectional view of the clamping assembly shown in FIG. 8 positioned about a tubular member in an open or non-clamped configuration;

FIG. 10 is a side cross-sectional view of the clamping assembly shown in FIG. 9 positioned about a tubular member in a closed or clamped configuration;

FIG. 11 is an alternatively disclosed clamping assembly positioned about a tubular member in an open or non-clamped configuration;

FIG. 12 is a side cross-sectional view of the clamping assembly shown in FIG. 11 positioned about a tubular member in the closed or clamped configuration;

FIG. 13 is a side perspective view of another embodiment of a clamping assembly with parts separated;

FIG. 14 is a side cross-sectional view of the clamping assembly of FIG. 13 positioned about a tubular member in a closed or clamped configuration;

FIG. 15 is a side perspective view, in partial cross-section, of an embodiment of the presently disclosed clamping assembly positioned about a tubular member in an open or non-clamped configuration;

FIG. 16 is a side perspective view, in partial cross-section, of the clamping assembly shown in FIG. 15 positioned about a tubular member in a closed or clamped configuration;

FIG. 17 is a side perspective view of another embodiment of the presently disclosed clamping assembly;

FIG. 18 is a cross-sectional view of the retention member of the clamping assembly shown in FIG. 17 shown along line 18-18;

FIG. 19 is a side view of an alternate embodiment of the presently disclosed clamping assembly;

FIG. 20 is a side plan view of yet another embodiment of the presently disclosed clamping assembly;

FIG. 21 is a side perspective view of the clamping assembly of FIG. 20;

FIG. 22 is a side perspective view of the clamping assembly of FIG. 20 positioned about a tubular member; and

FIG. 23 is a perspective view of another embodiment of the presently disclosed clamping assembly.

### DETAILED DESCRIPTION OF EMBODIMENTS

Embodiments of the presently disclosed clamping assemblies will now be described in detail with reference to the drawings wherein like reference numerals designate identical or corresponding elements in each of the several views. In the drawings and in the description which follows, the term "proximal" or "trailing," as is tradition, will refer to the end of the apparatus of the present disclosure which is closest to the operator (e.g., a clinician), while the term "distal" or "leading" will refer to the end of the apparatus which is furthest from the operator. As used herein, the term "subject" refers to a human patient or other animal. The term "clinician" refers to a doctor, nurse, or other care provider and may include support personnel.

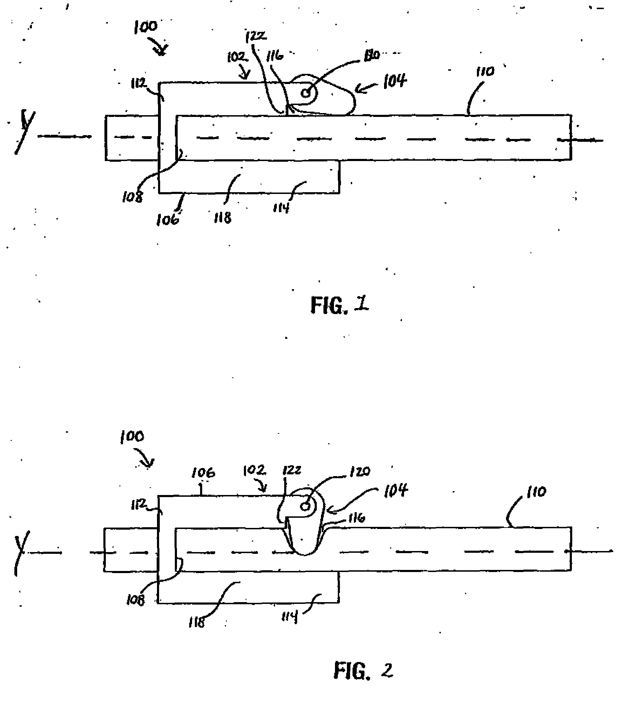

Referring to FIGS. 1 and 2, clamping assembly 100 includes a housing 102 and a clamp member 104. Housing 102 includes a body portion 106 which defines a substantially cylindrical throughbore 108 extending along longitudinal axis "y" of housing 102. The throughbore 108 is dimensioned to receive a resilient tubular member 110. Tubular member 110 can be any flexible or resilient fluid carrying conduit for medical or non-medical use. For example, tubular member 110 can be an extension tube of a catheter assembly or an intravenous (IV) line. Further, the housing 102 may be a hub of a catheter assembly, or it may be a separate component on the extension tube. Body portion 106 includes a proximal end portion 112 which can be adapted to frictionally engage tubular member 110, a distal end portion 114 including an opening 116 to allow passage of tubular member 110 therethrough, and sidewalls 118 dimensioned for passage and reception of tubular member 110 therein. The portion of the tubular member 110 which extends from the proximal end portion 112 of the housing 102 may be adapted for connection with various medical devices, such as a hemodialyzer, and the portion of tubular member 110 extending from the distal end 114 of housing 102 may be adapted for connection to a catheter which may be positioned within a subject. However, while the clamping assembly 100 is being described in this particular configuration, it is understood that the clamping assembly 100 may be flipped end-for-end and still work for its intended purpose.

The clamp member 104 is pivotably connected proximate to distal end portion 114 of housing 102 via pivot member or pin 120 such that clamp member 104 may freely rotate about pin 120. It should be understood that clamp member 104 may be coupled to the housing 102 via any suitable pivoting mechanism, such as hinges, joints, and other mechanical means within the purview of those skilled in the art. Clamp member 104 may also be joined with any other portion of the housing 102 as shown and discussed in other embodiments below. Clamp member 104 is configured to move between a first, open position which does not obstruct throughbore 108 and a second, closed position in which clamp member 104 projects into throughbore 108.

In the first position, clamp member 104 is positioned externally of the throughbore 108 such that the throughbore 108 is open for receiving the tubular member 110 and allowing the flow of fluids therethrough. As illustrated in FIG. 1, in the first position the clamp member 104 is oriented substantially parallel to the longitudinal axis "y." In the second position, clamp member 104 extends at least partially into the throughbore 108. As illustrated in FIG. 2, in the second position the clamp member 104 is oriented substantially perpendicular or transverse to the longitudinal axis "y" such that it obstructs the opening 116 of distal end 114 of housing 102 and engages tubular member 110 to inhibit the flow of fluid through tubular member 110. Housing 102 may include a stop 122 for preventing over-rotation of the clamp member 104 within throughbore 108.

In use, tubular member 110 is initially inserted through throughbore 108 of housing 102 while clamp member 104 is situated in the first, open position. In the first position, fluid can freely flow through tubular member 110. To inhibit fluid flow through tubular member 110, clamp member 104 may be moved, via manual manipulation or with an appropriate tool, to the second, closed position. While clamp member 104 is located in the second position, clamp member 104 compresses tubular member 110, thus significantly hindering or preventing fluid flow through tubular member 110.

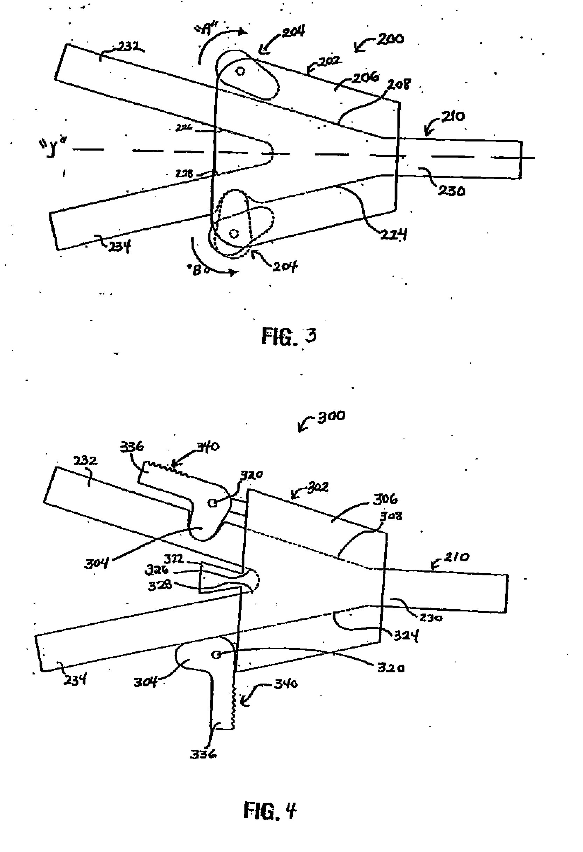

In another embodiment illustrated in FIG. 3, clamping assembly 200 includes a housing 202 having a body portion 206 including a throughbore 208 which may be bifurcated along a portion thereof. The clamping assembly 200 may accommodate one or more tubular members, such as a y-shaped tubular member 210. Throughbore 208 includes a central segment 224 extending along longitudinal axis "y," which divides into two branch segments 226 and 228 at an angle to longitudinal axis "y." Y-shaped tubular member 210 includes a single distal tube portion 230 and two proximal tube portions 232 and 234 which are configured for placement within throughbore 208. The distal tube portion 230 and the proximal tube portions 232 and 234 define a split fluid flow path through the tubular member 210 that is capable of independent fluid control. For example, distal tube portion 230 may be a dual lumen catheter in which each of proximal tube portions 232 and 234 are fluidly connected with one of the lumens defined in the distal tube portion 230. As both proximal tube portions 232 and 234 utilize distal tube portion 230, each of the proximal tube portions 232 and 234 can receive or remit fluid through the distal tube portion 230.

In embodiments, the distal tube portion 230 of the y-shaped tubular member 210 is adapted for insertion into a subject such that blood may be withdrawn through one of the proximal tube portions 232 or 234 which is connected to a hemodialysis unit for removal of waste and toxins from the blood, such as a dual lumen catheter. After purification, the dialyzed blood is returned to the subject via the other proximal tube portion 232 or 234. Accordingly, one proximal tube portion may be utilized for blood removal and the other for blood return. In other embodiments, both proximal end portions 232 and 234 may be utilized together for flow in the same direction, such as for delivery of IV fluids from two different sources. Further, the housing 202 may be the hub of a catheter.

Clamp members 204, which are substantially similar to clamp member 104 of FIGS. 1 and 2, are utilized for permitting or blocking the flow of fluid through throughbore 208. Each branch segment 226 and 228 of throughbore 208 has a clamp member 204 associated therewith for independent opening or closing of the proximal tube portion 232 or 234 positioned therein. Clamp member 204 may be moved between a first, open position to a second, closed position in the direction of arrows "A" and "B" and may be returned from the second, closed position to the first, open position in the opposite direction. Like clamp member 104, clamp member 204 permits the flow of fluids through its respective proximal tube portion 232 or 234 of throughbore 208 when placed in the first position and obstructs the flow of fluid therethrough when placed in the second position as the clamp member 204 engages its respective proximal tube portion 232 or 234 and pinches or crimps the tube closed. Housing 202 may include a stop, such as shown in FIG. 4, for preventing flexure of the proximal tube portions 232 and 234 away from clamp member 204 during clamping.

Turning now to FIG. 4, a clamping assembly 300 of the present disclosure includes a clamp actuator 336 for positioning of a clamp member 304. Clamping assembly 300 includes a housing 302 having a body portion 306 including a throughbore 308 having a central segment 324 which splits into two branch segments 326 and 328. Clamp actuator 336 may be monolithically formed with, or separately attached to, clamp member 304, which is operably connected to housing 302 and rotates about a pivot member or pin 320. Clamp actuator 336 may be any mechanism within the purview of those skilled in the art capable of moving clamp member 304 between a first, open position and a second, closed position. As illustrated in the current embodiment, clamp actuator 336 is a lever including an elongated body which facilitates grasping by a clinician to effect movement of the clamp member 304 into and out of through bore 308. Clamp actuator 336 may include a contoured outer surface 340 which may include a rugged texture to increase the friction and grip between the clinician's fingers and the clamp actuator 336.

The clamp member 304 associated with branch segment 328 of throughbore 308 is shown positioned in the first, open position thereby permitting fluid flow through proximal tube portion 234 and thus distal tube portion 230 of tubular member 210. The clamp member 304 associated with branch segment 326 of throughbore 308 is shown positioned in the second, closed position thereby engaging proximal tube portion 232 of tubular member 210 and obstructing fluid flow therethrough. During operation, fluid flow through either of proximal tube portions 232 and 234 may be promoted or inhibited by moving the respective clamp member 304 between these first and second positions. A stop 322 may be provided for preventing movement of the proximal tube portions 232 and 234 away from clamp member 304 during clamping.

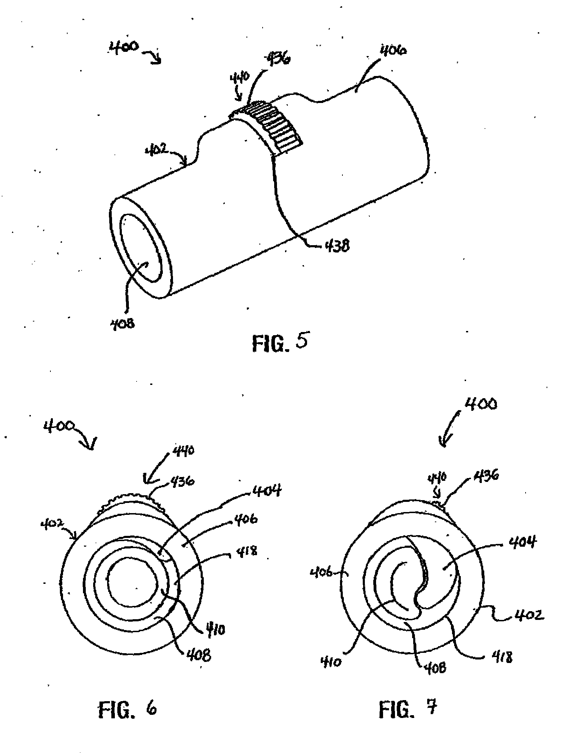

FIGS. 5-7 illustrate another embodiment of a clamping assembly 400 of the present disclosure including a clamp actuator 436 in the form of a slide knob. Clamping assembly 400 includes a housing 402 having a body portion 406 which includes sidewall 418 defining a substantially cylindrical throughbore 408 which is configured to receive extension tube 410. The housing 402 may be part of the hub of a catheter, or may be a separate component. Sidewall 418 of housing 402 defines a slot 438 for positioning of clamp actuator or slide knob 436 therein. Clamp actuator 436 is operably connected with clamp member 404 which is disposed within sidewall 418 of housing 402. Clamp member 404 is pivotably connected to the sidewall 418 such that clamp member 404 pivots into and out of through bore 408 upon actuation of clamp actuator 436.

Clamp actuator 436 may be moved from a first setting corresponding to a first, open position of throughbore 408 as illustrated in FIG. 6 to a second setting corresponding to a second, closed position of through bore 408 as illustrated in FIG. 7. In the first position, clamp member 404 is substantially aligned with sidewall 418 and does not obstruct throughbore 408. In the second position, clamp member 404 blocks at least part of throughbore 408 and compresses extension tube 410 located within throughbore 408, thereby hindering fluid flow through the extension tube 410.

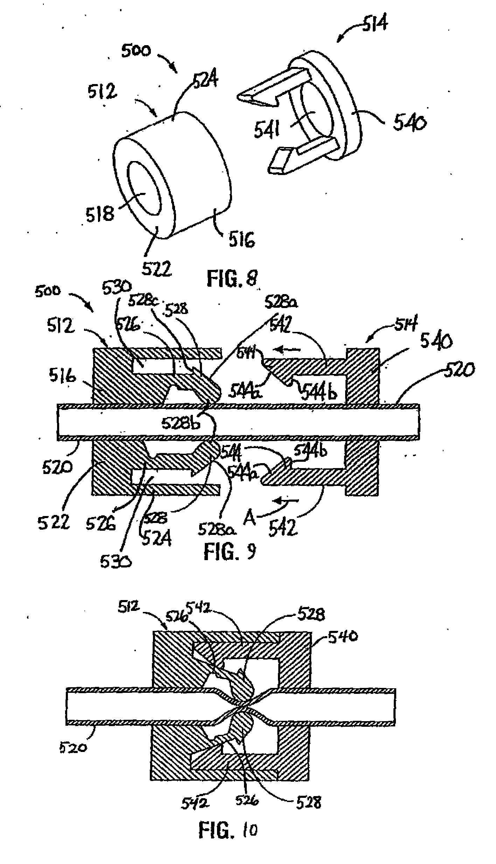

Referring now to FIGS. 8 and 9, a clamping assembly 500 in accordance with the present disclosure includes a clamp member 512 and a clamp actuator 514. Clamp member 512 includes a substantially cylindrical body 516 which defines a throughbore 518 dimensioned to receive a resilient tubular member 520, e.g., an extension tube of a catheter assembly. Body 516 includes a base 522 and an annular sidewall 524. A pair of clamp arms 526 extend from base 522 in cantilevered fashion within sidewall 524 on opposite sides of through bore 518. Each clamp arm 526 is formed with an engagement member 528 on its end opposite base 522 and is inwardly spaced from sidewall 524 to define a recess 530. Each engagement member 528 includes an angled surface 528a, an atraumatic clamping surface 528b which is positioned to engage tubular member 520, as will be discussed in further detail below, and a shoulder 528c.

Clamp actuator 514 includes a base member 540 defining a throughbore 541 and a pair of diametrically opposed actuator arms 542 which extend from base member 540. Each actuator arm 542 has an actuator head 544 positioned on its end opposite base member 540. Each actuator head 544 includes a tapered or angled face 544a which is configured and positioned to engage angled surface 528a of engagement member 528 of clamp member 512 when clamp member 512 and clamp actuator 514 are assembled, as will be discussed in further detail below. Actuator head 544 also defines a shoulder 544b positioned between actuator head 544 and base member 540.

Referring to FIGS. 9 and 10, in use, a tubular member 520 is positioned through throughbore 518 of clamp member 512 and through throughbore 541 of clamp actuator 514. Clamp actuator 514 is moved along tubular member 520 towards clamp member 512 in the direction indicated by arrow A in FIG. 9 such that actuator arms 542 move into recess 530 of clamp member 512. When angled face 544a of actuator head 544 of clamp actuator 514 engages angled surface 528a of engagement member 528, clamp arms 526 of clamp member 512 are cammed inwardly towards tubular member 520 as shown in FIG. 10 such that atraumatic clamping surfaces 528b compress tubular member 520 therebetween to obstruct and/or prevent flow through tubular member 520. When shoulder 544b of actuator head 544 passes over angled surface 528a of engagement member 528, shoulder 528c moves into alignment with shoulder 544b of actuator head 544 to prevent separation of clamp actuator 514 from clamp member 512 until a predetermined force in a direction opposite to arrow A is applied to clamp actuator 514 or until the clamp actuator 514 is rotated relative to clamp member 512 to move the actuator head 544 out of alignment with the engagement members 528.

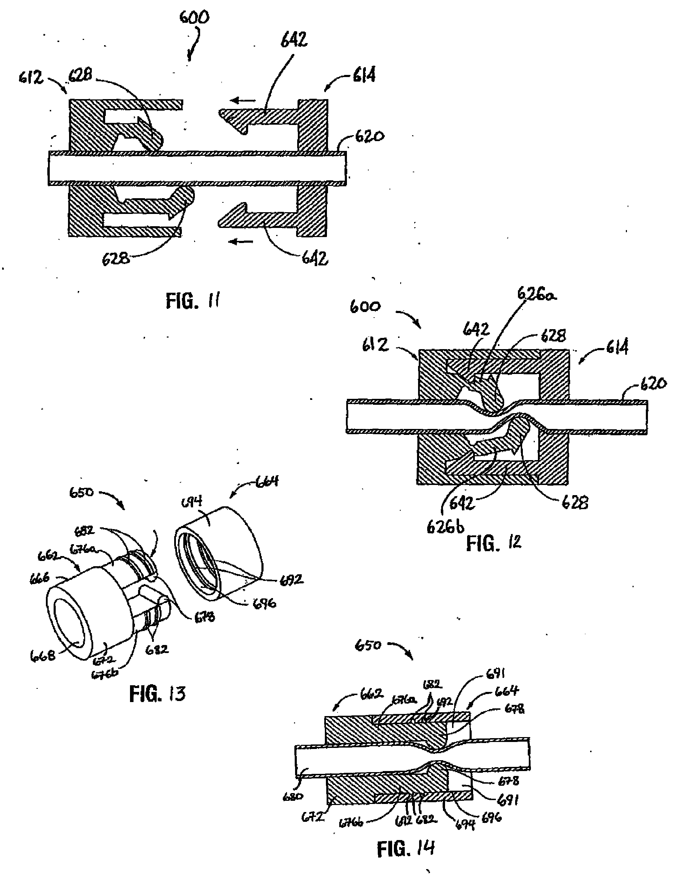

As indicated in FIGS. 9 and 10, clamp arms 526 are of equal length and tubular member 520 is compressed directly between atraumatic clamping surfaces 528b. In an alternative embodiment shown in FIGS. 11 and 12, clamping assembly 600 includes a clamp member 612 which has clamp arms 626a and 626b of different lengths such that tubular member 620 is twisted between engagement members 628 of clamp arms 626a and 626b. Clamp actuator 614 including actuator arms 642 is substantially as described above with respect to clamp actuator 514 and will not be described in further detail herein.

In yet another embodiment illustrated in FIGS. 13 and 14, clamping assembly 650 includes a clamp member 662 and a clamp actuator 664. The clamp member 662 includes a substantially cylindrical body 666 defining a throughbore 668 that is dimensioned to receive a tubular member 680. Body 666 includes a base 672 and a pair of clamp arms 676a and 676b extending from the base 672. Each clamp arm 676 is formed with an engagement member 678 on its end opposite base 672 for engagement with a tubular member 680 and at least one ridge 682 for releasable engagement with clamp actuator 664. Clamp actuator 664 includes an annular wall 694 having an inner surface 696 defining a throughbore 691. The inner surface 696 includes at least one slot 692 dimensioned to receive ridge 682 of clamp arm 676 to secure clamp member 662 and clamp actuator 664 together.

In use, tubular member 680 is positioned through throughbore 668 of clamp member 662 and throughbore 691 of clamp actuator 664. Clamp actuator 664 is moved along tubular member 680 towards clamp member 662 such that clamp arms 676 are received within annular wall 694 of clamp actuator 664 until the ridges 682 of clamp arm 676 lock into slot 692 of inner surface 696. Annular wall 694 cam the clamp arms 676 of clamp member 662 inwardly towards tubular member 680 as shown in FIG. 14 such that engagement member 678 compress tubular member 680 therebetween to obstruct and/or prevent flow through tubular member 680.

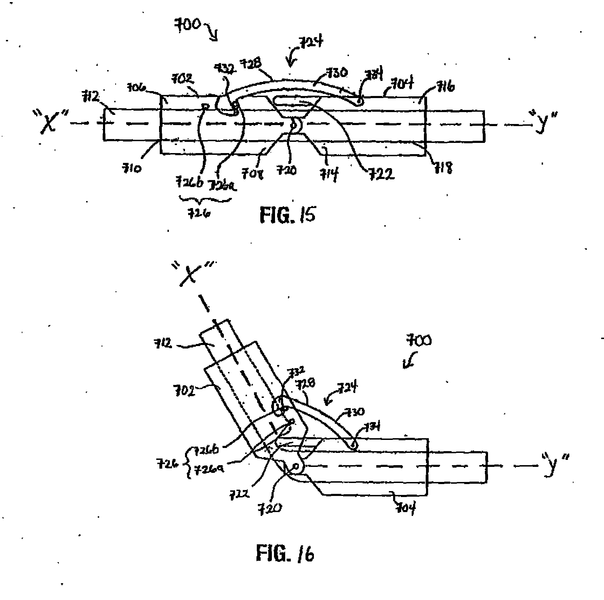

Turning now to FIGS. 15 and 16, an alternate mechanism for allowing or blocking fluid flow through tubular members is illustrated. A clamping assembly 700 in accordance with the present disclosure includes a first housing 702 operably connected to a second housing 704. First housing 702 defines a longitudinal axis "x" extending along a length of the first housing 702 from a proximal end 706 to a distal end 708. First housing 702 further defines an internal, substantially cylindrical, longitudinal passageway 710 dimensioned to receive a tubular member 712.

Second housing 704 defines a longitudinal axis "y" extending along a length of the second housing 704 from a proximal end 714 to a distal end 716. Second housing 704 also defines an internal, substantially cylindrical, longitudinal passageway 718 dimensioned to receive tubular member 712. In the illustrated embodiment of FIG. 15, longitudinal axis "y" of the second housing 704 is coincident with axis "x" of first housing 702, thereby aligning longitudinal passageway 718 of second housing 704 with the longitudinal passageway 710 of first housing 702. This position does not inhibit flow of fluids through the tubular member 712 and thus defines a first, unclamped position of the clamping assembly 700.

First housing 702 and second housing 704 are joined via a pivot member 720. Second housing 704 may include an engagement member 722 in the form of a rigid elongate body extending from proximal end 714 of the second housing 704. Engagement member 722 extends along an axis which is substantially parallel to longitudinal axis "y" of the second housing 704 beyond pivot member 720 without contacting first housing 702 when the first housing 702 and the second housing 704 are positioned in the first, unclamped position.

The first and second housings 702 and 704 are maintained in the first, unclamped position via latch mechanism 724. Latch mechanism 724 is adapted for selectable, releasable positioning of the first housing 702 relative to the second housing 704. Latch mechanism 724 includes a first coupling segment 726 secured to first housing 702 and a second coupling segment 728, complimentary to the first coupling segment 726, secured to the second housing 704. The first and second coupling segments 726 and 728 may be secured to respective housings 702 or 704 by conventional means such as, for example, ultrasonic welding, with the use of adhesive, or by mechanical coupling means. Alternatively, the coupling segments 726 and 728 may be monolithically formed with respective housings 702 and 704. It is envisioned that the first and second coupling segments may be any of a variety of coupling means such as, for example, bayonet couplings, snap fit arrangements, frictional fittings, tongue and groove configurations, threaded arrangements, cam-lock mechanisms, and the like.

As illustrated in FIG. 15, second coupling segment 728 is a latch having a latch arm 730 pivotally connected to second housing 704 via joint 734 and a locking surface 732. The locking surface 732 may be a curved or angular portion, such as a hook, adapted for secure, yet releasable engagement with first coupling segment 726. First coupling segment 726 is configured to include at least two contact points for engagement with the locking surface 732 of the second coupling segment 728. The contact points allow for variable positioning of the latch mechanism 724. As illustrated, first coupling segment includes a first pin 726a and a second pin 726b which extend from the surface of the first housing 702 and are configured to receive locking surface 732 of second coupling segment or latch 728.

The first housing 702 and the second housing 704 are maintained in a first, unclamped position when latch mechanism 724 is placed in the first engaged position by securing the locking surface 732 of the latch 728 over the first pin 726a of the first coupling segment 726. Latch mechanism 724 may be released by lifting latch arm 730 in a direction that is substantially transverse to the longitudinal axis "y" in order to overcome the holding force of the locking surface 732 with the first pin 726a, or by pivoting one of the first or second housings 702 or 704 thereby releasing the tension between the locking surface 732 and pin 726a, and allowing for free rotation of the latch 728 away from the first coupling segment 726.

As illustrated in FIG. 16, clamp assembly 700 may be placed in a second, clamped position when latch mechanism 724 is placed in the second engaged position through attachment of the locking surface 732 with the second pin 726b. To place locking surface 732 into engagement with second pin 726b, the first housing 702 and the second housing 704 are brought out of longitudinal alignment and bent at an angle relative to each other so that the locking surface 732 of latch 728 may be fastened against second pin 726b, thereby holding the first and second housings 702 and 704 in an angular relationship with respect to each other. The angle between the longitudinal axes of the first and second housings 702 and 704 may be an acute, obtuse, or right angle. The first and second housings 702 and 704 are pivoted in relation to each other to move the latch mechanism 724 between the disengaged position and the engaged position.

When the longitudinal axes "x" and "y" of the first housing 702 and the second housing 704, respectively, are positioned to define an angular relationship, tubular member 712 which is disposed therein will bend or kink to obstruct the flow of fluid therethrough. The degree of obstruction will depend on factors such as the angle defined between the "x" and "y" axes, the diameter of the tubular member, and the resiliency of the material of the tubular member. In addition, the engagement member 722 of the second housing 704 may be at least partially positioned within the passageway 710 of the first housing 702 when the first and second housings 702 and 704 are in the second engaged position such that the engagement member 722 compresses tubular member 712, thus obstructing or preventing fluid flow through tubular member 712.

Latch mechanism 724 may be released by sliding latch 728 away from the first housing 702 thereby overcoming the holding force of the locking surface 732 with second pin 726b or by further pivoting the first and/or second housings 702 or 704 towards each other to release the tension between the locking surface 732 and the second pin 726b thereby allowing for free rotation of the second coupling segment 728 away from the first coupling segment 726. Thus, the release of latch mechanism 724 from second pin 726b enables the movement of the clamp assembly 700 between the first, unclamped position and the second, clamped position.

The placement of the first and second coupling segments 726 and 728 may be reversed such that the first coupling segment 726 of first housing 702 may be in the form of a latch while the second coupling segment 728 of second housing 704 may be in the form of pins. Other fastening configurations are also envisioned such as the use of a latch with slots or notches formed in a housing, or variable contact points on the latch arm, such as two locking surfaces longitudinally spaced along the latch arm.

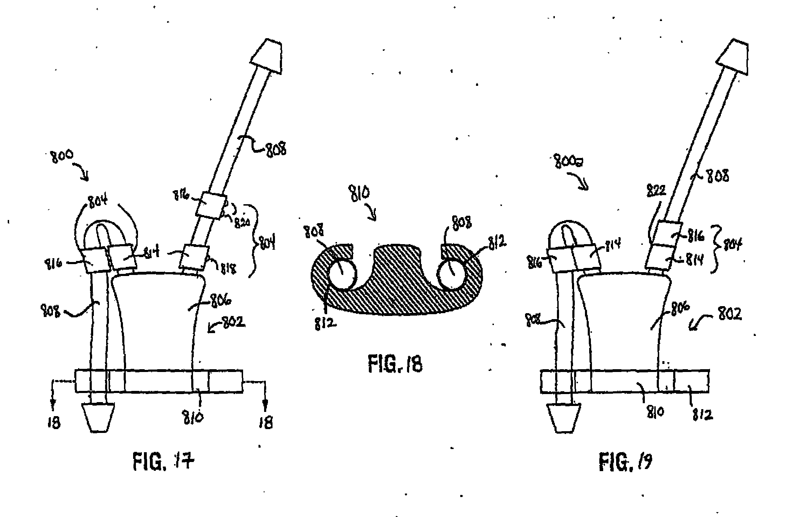

FIGS. 17-18 illustrate yet another alternate embodiment of a clamping assembly 800 in accordance with the present disclosure. Clamping assembly 800 includes a hub 802 and a locking mechanism 804. The hub 802 includes a body portion 806 and a base portion 810. Body portion 806 defines one or more throughbores (not shown) and supports one or more tubular members 808 which communicate with the throughbores. Base portion 810 defines at least one opening 812 dimensioned to releasably receive a tubular member 808 when the tubular member 808 is positioned in a curved or bent configuration. As illustrated in the current embodiment, clamping assembly 800 includes a hub 802 having a body portion 806 supporting two tubular members 808 and a base portion 810 defining two openings 812, each opening being dimensioned to individually receive a tubular member 808.

Locking mechanism 804 includes first and second sleeves 814 and 816 disposed around tubular member 808. As shown in the embodiment of FIG. 17, first sleeve 814 may include first teeth 818 and second sleeve 816 may include second teeth 820 which are configured to engage first teeth 818. The first and second sleeves 814 and 816 are maintained in a first, unlocked position when the first and second teeth 818 and 820 are not engaged and in a second, locked position by engaging the first teeth 818 with the second teeth 820.

In use, the tubular member 808 may be bent and placed within opening 812 of base portion 810 of hub 802. Sleeves 814 and 816 may be placed in the locked position by approximating and engaging first and second teeth 818 and 820. The locked position secures the tubular member 808 in a bent position, thereby forming a kink in the tubular member 808 and obstructing fluid flow therethrough. The locking mechanism 804 may then be disengaged and returned to the unlocked position so that tubular member 808 is no longer bent and fluid can freely flow therethrough.

It should be understood that any mechanism for securing sleeves 814 and 816 may be utilized to maintain the bent position of the tubular member 808. For example, clamping assembly 800a illustrated in FIG. 19 is substantially identical to clamping assembly 800 of FIG. 17, except that locking mechanism 804 includes a hinge 822 pivotally connecting first and second sleeves 814 and 816. Hinge 822 assists in maintaining tubular member 808 in the bent position, thereby facilitating the formation of a kink in tubular member 808 and hindering or preventing fluid flow therethrough.

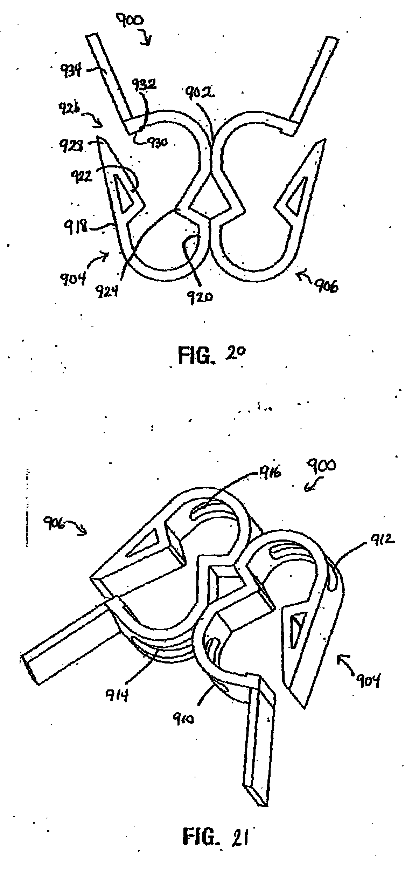

Turning now to FIGS. 20-22 another embodiment of a clamping assembly is illustrated. Clamping assembly 900 includes an integral, monolithic body 902 including a first clamping member 904 and a second clamping member 906. First and second clamping members 904 and 906 are disposed in a side by side relationship to each other as a mirror image pair. Each of the first and second clamping members 904 and 906 is configured to move between open and closed positions. As discussed in detail below, first and second clamping members 904 and 906 allow free flow of fluid through a tubular member 908 when placed in the open position and hinder the passage of fluid through a tubular member 908 when placed in the closed position.

First clamping member 904 defines proximal and distal openings 910 and 912 to allow passage of a tubular member 908 therethrough. Second clamping member 906 also defines proximal and distal openings 914 and 916 to allow passage of a tubular member 908 therethrough.

Since the structure and operation of second clamping member 906 is substantially identical to the structure and operation of first clamping member 904, the clamping member is discussed singularly with reference to the first clamping member 904.

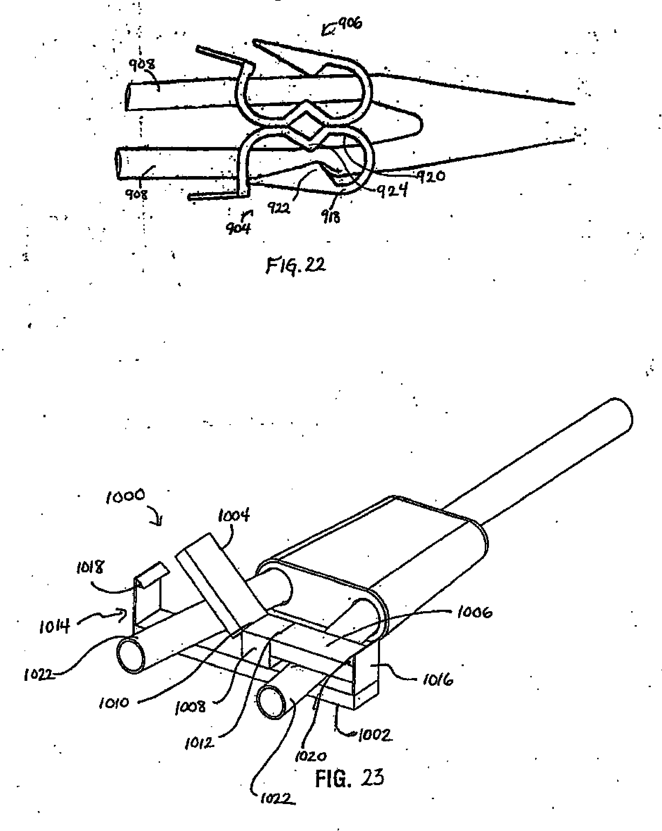

First clamping member 904 includes a movable portion 918 and a fixed portion 920. Movable and fixed portions 918 and 920 each include at least one protuberance 922 and 924, respectively. In use, protuberance 922 of movable portion 918 compresses tubular member 908 against protuberance 924 of fixed portion 920 when movable portion 918 is moved toward fixed portion 920, as shown in FIG. 22. In the depicted embodiment, both movable and fixed portions 918 and 920 have a protuberance 922 and 924 having a triangular shape, but those skilled in the art will recognize that each of movable and fixed portions 918 and 920 may have a plurality of protuberances 922 and 924 with any suitable configuration.

First clamping member 904 also includes a locking mechanism 926 for maintaining movable portion 918 and fixed portion 920 in the approximated position. Locking mechanism 926, which may be a snap-fit lock, incorporates a locking member 928 and a locking receiver 930. Locking member 928 is positioned on an end of movable portion 918, whereas locking receiver 930 extends from fixed portion 920. Locking receiver 930 may have a curved profile and includes an abutting surface 932 configured to hold locking member 928. In operation, locking member 928 snaps into locking receiver 930 and, as a result, abutting surface 932 maintains movable portion 918 approximated with fixed portion 920. When movable portion 918 is moved towards the fixed portion 920, protuberances 922 and 924 are also approximate with each other thereby compressing tubular member 908 and obstructing fluid flow therethrough. Locking receiver 930 further incorporates an actuation flange 934 for facilitating engagement and disengagement of locking member 928 from locking receiver 930. FIG. 20 illustrates clamping member 904 in the approximated position thereby obstructing fluid flow through tubular member 908 and clamping member 906 in a spaced apart position in which fluid flow through tubular member 908 is not obstructed.

FIG. 23 illustrates another embodiment of a clamping assembly 1000. Clamping assembly 1000 incorporates a monolithic support bar 1002, a first clamping jaw 1004, and a second clamping jaw 1006. A connecting member 1008 is secured to a central portion of the support bar 1002 and first and second clamping jaws 1004 and 1006 are pivotally coupled to connecting member 1008. In one embodiment, a first hinge 1010 pivotally connects first clamping jaw 1004 to connecting member 1008, and a second hinge 1012 pivotally secures second clamping jaw 1006 to connecting member 1008. Each of first and second clamping jaws 1004 and 1006 are movable between open and closed positions. In the open position, first and second clamping jaws 1004 and 1006 are each spaced apart from support bar 1002. In the closed position, first and second clamping jaws 1004 and 1006 are each in juxtaposed alignment with support bar 1002. Clamping assembly 1000 further includes first and second locking members 1014 and 1016 located on opposite ends of support bar 1002. During operation, locking members 1014 and 1016 secure first and second clamping jaws 1004 and 1006, respectively, in the closed position. In the depicted embodiment, each locking member 1014 and 1016 is a snap fit locking device that includes an abutment wall 1018 and 1020 configured to engage the corresponding clamping jaw 1004 and 1006. Alternatively, other known locking structure can be provided.

During operation, the first and second clamping jaws 1004 and 1006 are placed in an open position. (See e.g., clamping jaw 1004). Then, one tubular member 1022 is positioned between support bar 1002 and first clamping jaw 1004 and another tubular member 1022 is positioned between support bar 1002 and second clamping jaw 1006. After positioning tubular members 1022 in the appropriate locations, first and second clamping jaws 1004 and 1006 may be moved to a closed position. (See, e.g., clamping jaw 1006). As the first and second clamping jaws 1004 and 1006 are moved toward support bar 1002, first and second clamping jaws 1004 and 1006 compress tubular members 1022, thus obstructing fluid flow through tubular members 1022.

It will be understood that various modifications and changes in form and detail may be made to the embodiments of the presently disclosed clamping assemblies without departing from the spirit and scope of the disclosure. Therefore, the above description should not be construed as limiting, but merely as exemplifications of embodiments thereof. Those skilled in the art will envision other modifications within the scope and spirit of the present disclosure.

## Claims

1. A clamping assembly comprising:
a housing including a proximal end portion and a distal end portion, the housing defining a throughbore dimensioned to receive a tubular member; and
at least one clamp member pivotally coupled to the housing, the at least one clamp member being configured to move between a first position positioned externally of the throughbore to allow fluid flow through a tubular member positioned within the throughbore and a second position extending into the throughbore and into contact with a tubular member to obstruct fluid flow through the tubular member positioned within the throughbore.

2. The clamping assembly of claim 1, wherein the clamp member is pivotally connected to the distal end portion of the housing.

3. The clamping assembly of claim 1, wherein the clamp member is pivotally connected to the proximal end portion of the housing.

4. The clamping assembly of claim 1, wherein the clamp member is pivotally connected between the proximal and distal end portions of the housing.

5. The clamping assembly of any one of claims 1 to 4, wherein the housing further comprises a stop to prevent over-rotation of the clamp member within the throughbore.

6. The clamping assembly of any one of the preceding claims, wherein the throughbore includes a central segment extending along a portion of the housing which divides into two branch segments.

7. The clamping assembly of claim 6, wherein two clamp members are pivotally connected to the housing such that each of the two branch segments has one of the two clamp members associated therewith for independent movement of the clamp members between the first and second positions to control fluid flow through a portion of a y-shaped tubular member positioned within the respective branch segment of the throughbore.

8. The clamping assembly of any one of the preceding claims, further comprising a clamp actuator for moving the clamp member between the first and second positions.

9. The clamping assembly of claim 8, wherein the clamp actuator is monolithically formed with the clamp member.

10. The clamping assembly of any one of claims 8 to 9, wherein the clamp actuator includes a contoured outer surface to facilitate gripping of the clamp actuator.

11. The clamping assembly of any one of claims 8 to 10, wherein the clamp actuator is disposed within a slot formed in a sidewall of the housing.

12. The clamping assembly of any one of the preceding claims, wherein the housing is a hub of a catheter.

13. A fluid control system comprising:
a tubular member defining a channel extending therethrough for passage of a fluid;
a housing defining a throughbore dimensioned to receive the tubular member; and
a clamp member pivotally connected to the housing, the clamp member being positioned and configured to rotate into the throughbore of the housing to compress the tubular member when the tubular member is positioned therein and to rotate out of the throughbore of the housing to uncompress the tubular member when the tubular member is positioned therein.

14. The fluid control system of claim 13, wherein the clamp member is positioned to rotate into the throughbore to a position which is substantially transverse to a longitudinal axis of the throughbore.

15. The fluid control system of any one of claims 13 to 14, wherein the clamp member is positioned to rotate out of the throughbore to a position which is substantially parallel to a longitudinal axis of the throughbore.

16. The fluid control system of any one of claims 13 to 15, further comprising a clamp actuator for rotating the clamp member into and out of the throughbore of the housing.

17. The fluid control system of claim 16, wherein the clamp actuator is a lever attached to the clamp member.

18. The fluid control system of any one of claims 13 to 17, wherein the tubular member is an extension tube of a catheter assembly.
